# EUROPÄISCHE PATENTSCHRIFT

(11) **EP 3 165 124 B1**
(45) Veröffentlichungstag und Bekanntmachung des Hinweises auf die Patenterteilung: **20.11.2019**
(21) Anmeldenummer: 16197193.2
(22) Anmeldetag: 04.11.2016
(51) Int. Cl.: A45D 34/04, A61B 17/54, A61B 17/20, A61M 35/00, A61B 17/00, A61B 17/32

(54) **DERMABRASIONSSTICK**
DERMAL ABRASION STICK
STICK D'ABRASION DERMALE

(30) Priorität: 09.11.2015 DE 102015221977
(43) Veröffentlichungstag der Anmeldung: 10.05.2017
(73) Patentinhaber: Bauer, Gwenaël, 67000 Strasbourg (FR)
(72) Erfinder: Lippert, Torsten Herbert, 77656 Offenburg (DE)
(74) Vertreter: Kohler Schmid Möbus Patentanwälte

(56) Entgegenhaltungen:
- EP-A1- 1 138 221
- US-A- 2 924 219
- US-A1- 2004 185 067
- US-A1- 2013 345 723
- US-B1- 8 161 983
- US-B2- 8 128 638

## Beschreibung

Die Erfindung betrifft ein stabförmiges Dermabrasionswerkzeug mit einem Handteil zum Halten und Führen des Dermabrasionswerkzeugs und einem an einem axialen Ende des Dermabrasionswerkzeugs vorgesehenen Dermabrasionsbereich, wobei der Dermabrasionsbereich eine Trägermatrix aufweist, an der mehrere Reibvorsprünge zur mechanischen Hautbehandlung angeordnet oder ausgebildet sind, wobei die Reibvorsprünge jeweils eine maximale Querschnittsbreite zwischen 0,5 µm und 500 µm aufweisen.

Bei der Dermabrasion handelt es sich um ein Verfahren zur Abtragung der obersten Hautschicht bzw. oberer Hautschichten. Sie kann bei Verhornung der Haut, zur Behandlung von Narben, zur Entfernung oberflächlicher Tätowierungen, bei Pigmentveränderungen der obersten Hautschichten und/oder bei gutartigen Hautveränderungen angewendet werden. Dermabrasion- erhöht die Aufnahmefähigkeit/Einbringung von Wirkstoffen in die Haut;- reduziert Falten durch einen (Ab-)Schleifeffekt;- induziert die Hauterneuerung (Regeneration).

Zur Hautabtragung werden Dermabrasionswerkzeuge eingesetzt, wobei die Hautabtragung typischerweise durch sich mechanisch bewegende Reibflächen, durch Elektroden oder durch Lichteinstrahlung unterstützt oder bewirkt wird.

Aus der US2004/185067 ist ein stabförmiges Dermabrasionswerkzeug, wobei das Handteil einen Hohlraum aufweist, und eine Pumpe welche fluidisch mit dem Hohlraum verbunden ist, bekannt. Die zweiteilige Form des Patentanspruchs 1 ist basiert auf diesem Dokument.

Aus der EP 1 764 010 A1 ist ein gattungsgemäßes Dermabrasionswerkzeug in Form eines Hautbearbeitungswerkzeugs zum Hautpeeling an einem Elektrohandgerät des persönlichen Bedarf bekannt geworden. Das bekannte Hautbearbeitungswerkzeug ist auf einen Elektrorasierer oder dergleichen aufsetzbar.

Die AU 2004229051 A1 offenbart ein manuell führbares Handteil eines Dermabrasionswerkzeugs, wobei das Handteil über einen Schlauch mit einer Vakuumpumpe verbunden ist.

Aus der US 8,128,638 B2 ist ein Dermabrasionswerkzeug in Form eines Handgeräts bekannt geworden. Ein Schleifkopf des Dermabrasionswerkzeugs wird im Betrieb rotiert und axial bewegt.

Die US 2015/0051620 A1 offenbart ein elektrisches Dermabrasionswerkzeug mit einer Batterie-betriebenen Luftpumpe.

Aus der US 2004/0143274 A1 ist ein Dermabrasionswerkzeug bekannt geworden, das einen Oberflächenbereich zur teilweisen Hautabtragung, ein Ansaugsystem zum Anziehen der Haut zum Dermabrasionswerkzeug hin und eine Möglichkeit zur Verabreichung eines sterilen Fluids auf die Haut aufweist.

Die US 2004/0010268 A1 offenbart ein Dermabrasionswerkzeug mit einer rotierenden Bürste.

Aus der US 2004/0254588 A1 ist eine Vorrichtung zur Hautbehandlung bekannt geworden, die eine Ansaugeinheit aufweist. Die Ansaugeinheit umfasst einen Filter zur Abscheidung hautfremder, angesaugter Substanzen.

Die WO 2004/006790 A1 offenbart einen plattenförmigen Schwamm mit einem an dem Schwamm angeordneten Handgriff. Der Schwamm ist mit einer Reinigungsflüssigkeit getränkt.

Aus der US 6,730,098 B2 ist ein Dermabrasionswerkzeug mit einem Schleifkopf und einer getrennt vom Schleifkopf ausgebildeten Ansaugdüse bekannt geworden.

Die US 2004/0138680 A1 offenbart ein stabförmiges Dermabrasionswerkzeug mit einem Elektromotor und einer Ansaughaube. In der Ansaughaube ist eine Schleifspitze zur mechanischen Hautbehandlung angeordnet, die mit dem Elektromotor verbunden ist.

Weitere Dermabrasionswerkzeuge mit einem Vakuumkopf sind aus der EP 0 992 221 A2, der US 6,911 031 B2, der DE 41 02 684 A1, der WO 2014/136013 A1 und der US 6,500,183 B1 bekannt geworden.

Die WO 2009/104178 A2 und die WO 2006/080012 A1 offenbaren Dermabrasionswerkzeuge, bei der die teilweise Hautabtragung durch RF-Elektroden erfolgt.

Aus der WO 03/059144 A2, der US 8,945,104 B2, der WO 00/28910 A1 und der US 9,149,650 B2 sind Dermabrasionswerkzeuge bekannt geworden, bei denen die Hautbehandlung durch Licht aus künstlichen Lichtquellen im Dermabrasionswerkzeug, insbesondere durch Laserlichtquellen, unterstützt wird.

Die WO 2010/124346 A1 offenbart ein Dermabrasionswerkzeug mit Elektroden zur Hautbehandlung. Zusätzlich zu dem Dermabrasionswerkzeug ist ein Zerstäuber zum Auftragen einer Flüssigkeit auf die Haut vorgesehen. Die Flüssigkeit kann Hyaloronsäure beinhalten.

Schließlich ist aus der WO 2008/078332 A1 ein Kissen zur Hautbehandlung bekannt geworden. Das Kissen ist beutelförmig ausgebildet und mit Kräutern gefüllt.

Nachteilig an den bekannten Dermabrasionswerkzeugen ist, dass diese verhältnismäßig komplex aufgebaut sind. Hieraus resultieren hohe Anschaffungs- und Wartungskosten für die bekannten Dermabrasionswerkzeuge. Weiterhin ist mit der Komplexität der bekannten Dermabrasionswerkzeuge ein hoher Reinigungs- und Desinfektionsaufwand verbunden. Darüber hinaus müssen die bekannten komplexen Dermabrasionswerkzeuge durch Fachkräfte bedient werden. Eine Anwendung am eigenen Körper ist dadurch in den meisten Fällen nicht möglich.

Aufgabe der vorliegenden Erfindung ist es demgegenüber, ein signifikant vereinfachtes Dermabrasionswerkzeug bereit zu stellen.

Diese Aufgabe wird erfindungsgemäß durch ein Dermabrasionswerkzeug mit den Merkmalen des Patentanspruchs 1 gelöst. Die Unteransprüche geben zweckmäßige Weiterbildungen an.

Erfindungsgemäß wird somit vorgeschlagen, zur Abtragung der Haut ein manuell betätigtes stabförmiges Dermabrasionswerkzeug einzusetzen. Das Dermabrasionswerkzeug weist ein Handteil auf, mit dem es von mehreren Fingern umgriffen werden und geführt werden kann. Zumindest ein axialer Endbereich des Dermabrasionswerkzeugs ist in Form eines Dermabrasionsbereichs ausgebildet. Der Dermabrasionsbereich weist eine Trägermatrix auf, an der mehrere Reibvorsprünge angeordnet oder ausgebildet sind. Vorzugsweise weist das Handteil keine entsprechenden Reibvorsprünge auf. Die Reibvorsprünge weisen jeweils eine maximale Querschnittsbreite zwischen 0,5 µm und 500 µm auf. Mit anderen Worten weisen die Reibvorsprünge maximale Ausmaße zwischen 0,5 µm und 500 µm auf. Die Trägermatrix ist einteilig mit dem Handteil ausgebildet.

Der Erfindung liegt somit die einfache aber überraschende Erkenntnis zugrunde, dass zur Dermabrasion ein Stab mit einem Dermabrasionsbereich einsetzbar ist, wobei der Dermabrasionsbereich fest mit einem Handteil des Dermabrasionswerkzeugs verbunden ist und Reibvorsprünge zwischen 0,5 µm und 500 µm aufweist.

Ein solches Dermabrasionswerkzeug ist signifikant einfacher und somit kostengünstiger herstellbar als bekannte Dermabrasionswerkzeuge. Weiterhin wird durch die einteilige Ausbildung der Desinfektionsaufwand deutlich verringert.

Auch bezüglich der nachhaltigen Entwicklung (sustainable development) setzt die vorliegende Erfindung Standards, da hiermit auf Wegwerf-Aufsätze verzichtet werden kann.

Die Dermabrasion kann besonders effektiv durchgeführt werden, wenn eine starre Verbindung zwischen der Trägermatrix und dem Handteil vorliegt.

Die Handhabung des Dermabrasionswerkzeugs wird vereinfacht, wenn das Handteil und/oder der Dermabrasionsbereich einen kreisförmigen oder ovalen Querschnitt aufweisen. Das Handteil ist insbesondere zylinderförmig ausgebildet. Weiter bevorzugt sind Handteil und/oder Dermabrasionsbereich rotationssymmetrisch zur Längsachse des Dermabrasionswerkzeugs ausgebildet.

Das Dermabrasionswerkzeug weist in seiner Längserstreckung vorzugsweise eine Länge zwischen 3 cm und 20 cm, insbesondere eine Länge zwischen 5 cm und 10 cm auf.

Senkrecht zu seiner Längsachse weist das Dermabrasionswerkzeug vorzugsweise eine Breite zwischen 1 cm und 10 cm, insbesondere eine Breite zwischen 2 cm und 4 cm auf.

Eine besonders effektive Dermabrasion wird erreicht, wenn die Ausmaße der Reibvorsprünge zwischen 20 µm und 350 µm betragen.

Die Reibvorsprünge können einteilig mit der Trägermatrix ausgebildet sein. Beispielsweise können die Reibvorsprünge durch ein Sandstrahlverfahren in der Trägermatrix ausgebildet werden. Vorzugsweise sind jedoch die Reibvorsprünge in Form von Partikeln ausgebildet, wobei die Partikel teilweise in die Trägermatrix eingebettet sind. Die Rauigkeit des Dermabrasionsbereichs kann in diesem Fall durch die Wahl der entsprechenden Partikel besonders einfach und präzise erfolgen. Die Partikel können in Form medizinischer Sand-Kristalle, Diamantenstaub, Glaspartikel, Metallpartikel und/oder Kunststoffpartikel ausgebildet sein.

Handteil und Trägermatrix weisen - im Gegensatz zu einem Schwamm - vorzugsweise eine starre, nicht elastisch-saugfähige Oberfläche auf. Hierdurch kann eine effektive Dermabrasion bei gleichzeitig einfacher Reinigbarkeit des Dermabrasionswerkzeugs erfolgen.

In der Erfindung sind das Handteil und die Trägermatrix aus Glas ausgebildet. Glas ist inert gegenüber den meisten Chemikalien und dadurch besonders effektiv und gründlich reinigbar. Weiterhin erlaubt die Härte des Glases eine effektive Kraftübertragung vom Handteil auf den Dermabrasionsbereich und letztlich auf die Haut, wodurch die Dermabrasion besonders effektiv und sicher durchführbar ist.

Im Falle eine Trägermatrix aus Glas sind die in Form von Partikeln ausgebildeten Reibvorsprünge vorzugsweise in die Oberfläche der Trägermatrix eingebrannt. Auf den Einsatz von Klebstoff zur Befestigung der Partikel kann hierdurch verzichtet werden, wodurch die Partikel besonders fest in der Trägermatrix haften und dadurch nicht in die bei der Dermabrasion entstehenden Mikrowunden gelangen. Weiterhin erfolgt durch den Verzicht auf Klebstoff eine hypoallergene Abrasion.

Der Dermabrasionsbereich kann eine konvexe oder konkave Grundform aufweisen. Hierdurch passt sich der Dermabrasionsbereich leicht an unterschiedlich geformte Hautpartien an. Alternativ dazu kann der Dermabrasionsbereich eine asymmetrische Form aufweisen.

Das Handteil weist einen Hohlraum und eine mit dem Hohlraum verbundene Öffnung zur Befüllung des Hohlraums mit einem Fluid auf. Das Dermabrasionswerkzeug dient dadurch einerseits mit seiner Außenseite zur Hautabtragung und andererseits durch seinen Hohlraum als Träger eines Fluids. Erfindungsgemäß liegt auch dann noch ein Hohlraum vor, wenn dieser, insbesondere mit einer Creme, einem Fluid oder dergleichen gefüllt ist.

Der Hohlraum kann zumindest teilweise mit einem Fluid gefüllt sein. Das Fluid kann zur ästhetischen Hautbehandlung (Schälkur/Peeling) eine saure Flüssigkeit aufweisen. Insbesondere kann das Fluid eine Fruchtsäure und/oder eine Glykolsäure aufweisen. Alternativ oder zusätzlich dazu kann das Fluid eine alkalische (basische) Flüssigkeit, wie beispielsweise eine Lauge, aufweisen.

In weiter bevorzugter Ausgestaltung des Dermabrasionswerkzeugs ist das Dermabrasionswerkzeug in Form eines transdermalen Wirkstofflieferanten (transdermal drug delivery system (TDDS)) ausgebildet. Hierzu weist das Fluid in dem Hohlraum ein Medikament auf. Das Medikament kann in Form eines Impfstoffs, insbesondere eines Pathogens, ausgebildet sein. Die Verabreichung eines Impfstoffs durch das erfindungsgemäße Dermabrasionswerkzeug ist besonders vorteilhaft, da hierzu keine Nadel eingesetzt werden muss. Immunisierungen können somit auch in Regionen erfolgen, in denen kein medizinisches Personal zugegen ist, an Personen, die Injektionen fürchten. Massenimmunisierungen können auch in Industrieländern durch die einfache Home-Applikation einfacher und schneller erreicht werden. Aufgrund der Mikroverletzungen, die von dem Dermabrasionswerkzeug hervorgerufen werden, kann die Wirkstoffübertragung in die Haut dabei effektiver erfolgen als beispielsweise durch bekannte transdermale Pflaster.

Der Dermabrasionsbereich kann eine Durchgangsausnehmung von der Umgebung des Dermabrasionswerkzeugs zum Hohlraum aufweisen. Hierdurch kann ein im Hohlraum vorhandenes Fluid unmittelbar über den Dermabrasionsbereich abgegeben werden. Mit anderen Worten kann das Fluid aus dem Hohlraum während der Hautbehandlung durch die Durchgangsausnehmung auf die behandelte Haut bzw. die zu behandelnde Haut gelangen. Alternativ oder zusätzlich dazu können der Hohlraum und die Durchgangsausnehmung dazu genutzt werden, die Haut während der Hautbehandlung anzusaugen.

Eine besonders effektive Hautansaugung kann erfolgen, wenn das Dermabrasionswerkzeug ein Rückschlagventil aufweist, über das der Hohlraum zumindest teilweise evakuierbar ist. Eine mechanische Verkleinerung des Hohlraums kann dabei zur Luftausleitung aus dem Hohlraum über das Rückschlagventil in die Umgebung des Dermabrasionswerkzeugs genutzt werden. Wird der Hohlraum anschließend wieder vergrößert, blockiert das Rückschlagventil und es erfolgt ein Ansaugen über die Durchgangsausnehmung, wodurch die Haut zum Dermabrasionsbereich angezogen wird.

Das Handteil kann im Bereich des Hohlraums zumindest teilweise flexibel ausgebildet sein, um eine leichte manuelle Vergrößerung und Verkleinerung des Hohlraums herbeiführen zu können. Das Handteil kann dabei aus Silikon oder einem anderen elastischen Material ausgebildet sein.

Das Dermabrasionswerkzeug weist eine manuell betätigbare Pumpe auf. Die Pumpe kann zur Ausgabe eines Fluids, das im Dermabrasionswerkzeug gespeichert ist, ausgebildet sein.

Dabei ist die Pumpe fluidisch mit dem Hohlraum verbunden. Die Pumpe kann dabei zur Förderung eines im Hohlraum vorhandenen Fluids ausgebildet sein. Alternativ oder zusätzlich dazu kann die Pumpe zur zumindest teilweisen Evakuierung des Hohlraums ausgebildet sein, um das Dermabrasionswerkzeug über die zuvor beschriebene Durchgangsausnehmung an die Haut ansaugen zu können.

Die Fertigung des Dermabrasionswerkzeugs wird signifikant vereinfacht, wenn das Handteil ein erstes Gewinde aufweist, das mit einem zweiten Gewinde der Pumpe verbunden ist. Das erste Gewinde ist dabei vorzugsweise in Form eines Außengewindes und das zweite Gewinde in Form eines Innengewindes ausgebildet.

Weiter bevorzugt weist das Dermabrasionswerkzeug keine elektrische Spannungsquelle, keine Lichtquelle, keinen Anschluss für eine elektrische Spannung, keinen Anschluss für einen Lichtleiter, keinen Anschluss für eine Druckluftversorgung und/oder keinen Anschluss für eine Vakuum-Versorgung auf. Mit anderen Worten ist das Dermabrasionswerkzeug in diesem Fall rein manuell durch einen Benutzer bewegbar und besonders einfach und robust sowie leicht desinfizierbar und reinigbar ausgebildet.

Weitere Merkmale und Vorteile der Erfindung ergeben sich aus der nachfolgenden detaillierten Beschreibung mehrerer Ausführungsbeispiele der Erfindung, aus den Patentansprüchen sowie anhand der Figuren der Zeichnung, die erfindungswesentliche Einzelheiten zeigt.

Die in der Zeichnung gezeigten Merkmale der Dermabrasionssticks sind derart dargestellt, dass die erfindungsgemäßen Besonderheiten deutlich sichtbar gemacht werden können. Die verschiedenen Merkmale können je einzeln für sich oder zu mehreren in beliebigen Kombinationen bei Varianten der Erfindung verwirklicht sein.

Es zeigen:
- Figur 1: eine Seitenansicht eines ersten Dermabrasionswerkzeugs;
- Figur 2: eine Seitenansicht eines zweiten Dermabrasionswerkzeugs;
- Figur 3: eine teilweise geschnittene Seitenansicht eines dritten Dermabrasionswerkzeugs ;
- Figur 4: eine Seitenansicht eines vierten Dermabrasionswerkzeugs mit einer Pumpe und einem Dermabrasionskörper;
- Figur 5: eine teilweise geschnittene Seitenansicht des Dermabrasionskörpers aus Figur 4; und
- Figur 6: eine Seitenansicht eines fünften Dermabrasionswerkzeugs.

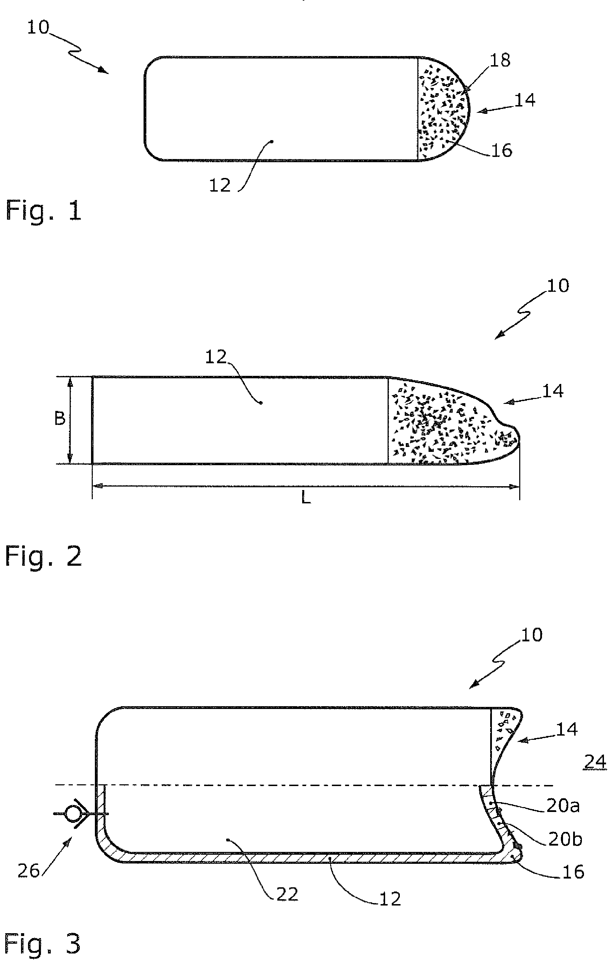

**Figur 1** zeigt ein Dermabrasionswerkzeug **10** in Form eines Dermabrasionssticks. Das Dermabrasionswerkzeug 10 ist in Form eines festen Körpers, insbesondere aus Glas, ausgebildet. Das Dermabrasionswerkzeug 10 weist ein Handteil **12** und einen Dermabrasionsbereich **14** auf. Das Handteil 12 ist zylinderförmig und der Dermabrasionsbereich 14 in Form einer Halbkugel ausgebildet. Das Handteil 12 ist einteilig mit einer Trägermatrix **16** des Dermabrasionsbereichs 14 ausgebildet. Der Dermabrasionsbereich 14 weist mehrere Reibvorsprünge **18** in Form von Partikeln auf, die fest in der Trägermatrix 16 verankert sind. Zur Hautbehandlung wird der Dermabrasionsbereich 14 manuell über die zu behandelnde Haut geführt, wobei die Reibvorsprünge 18 Mikrowunden in der Haut erzeugen.

**Figur 2** zeigt eine andere Ausführungsform eines Dermabrasionswerkzeugs 10. Auch das Dermabrasionswerkzeug 10 gemäß Figur 2 weist ein zylinderförmiges Handteil 12 auf. Ein sich am längsseitigen Ende des Dermabrasionswerkzeugs 10 befindende Dermabrasionsbereich 14 ist jedoch im Gegensatz zum Dermabrasionsbereich 14 gemäß Figur 1 asymmetrisch ausgebildet, um die Bearbeitung schwer erreichbarer Hautpartien zu erleichtern.

Das Dermabrasionswerkzeug 10 ist besonders kompakt und handlich ausgebildet, wenn es eine Länge L von weniger als 12 cm, insbesondere weniger als 9 cm aufweist.

Die Breite B des Dermabrasionswerkzeugs 10 beträgt bevorzugt weniger als 4 cm, insbesondere weniger als 3 cm. Die in den Figuren 1 und 2 gezeigten Dermabrasionswerkzeuge 10 bestehen nur aus einem Dermabrasionskörper, d.h. einem Handteil 12 und einem Dermabrasionsbereich 14.

**Figur 3** zeigt ein weiteres Dermabrasionswerkzeug 10 mit einem Dermabrasionsbereich 14. Eine Trägermatrix 16 des Dermabrasionsbereichs 14 weist Durchgangsausnehmungen **20a, 20b** auf, die einen Hohlraum **22** fluidisch mit der Umgebung 24 des Dermabrasionswerkzeugs 10 verbinden.

Ein Handteil 12 des Dermabrasionswerkzeugs 10 ist vorzugsweise zumindest teilweise flexibel ausgebildet. Bei einer Druckbeaufschlagung des Handteils 12 kann somit Luft aus dem Hohlraum 22 über die Durchgangsausnehmungen 20a, 20b in die Umgebung **24** entweichen. Lässt die mechanische Druckbeaufschlagung des Handteils 12 nach, entspannt sich das Handteil 12 und versucht in seine ursprüngliche Form zurück zu finden, wodurch es zu einem Ansaugeffekt durch die Durchgangsausnehmungen 20a, 20b in den Hohlraum 22 hinein kommt. Hierdurch kann zu behandelnde Haut bzw. behandelte Haut in den konkav ausgebildeten Dermabrasionsbereich 14 gezogen werden. Die Dermabrasionsbehandlung kann dadurch besonders effektiv erfolgen.

Der Ansaugeffekt in den Hohlraum 22 kann durch ein Rückschlagventil **26** verstärkt werden. Das Rückschlagventil 26 ist in Figur 3 lediglich in Form eines Schaltsymbols dargestellt. Das Rückschlagventil 26 sperrt eine Fluidbewegung in den Hohlraum 22 hinein und öffnet bei einer Fluidbewegung aus dem Hohlraum 22 hinaus. Mittels des Rückschlagventils 26 kann die Ansaugwirkung ohne Abnahme des Dermabrasionswerkzeugs 10 von der Haut während der Dermabrasionsbehandlung aufrecht erhalten werden.

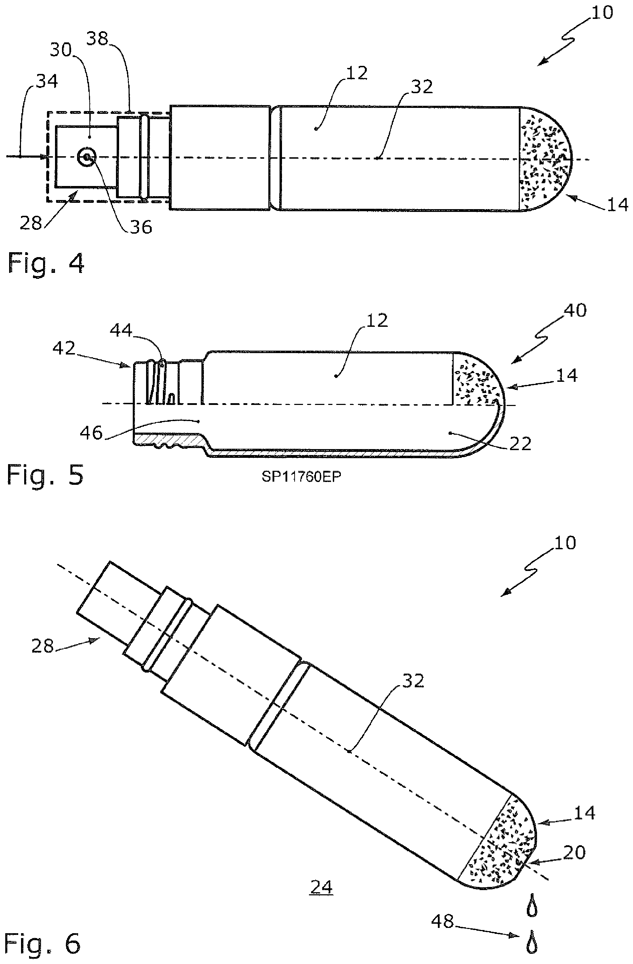

**Figur 4** zeigt ein weiteres Ausführungsbeispiel eines Dermabrasionswerkzeugs 10. Das Dermabrasionswerkzeug 10 weist ein Handteil 12 auf, das mit einer Pumpe **28** verbunden ist. Die Pumpe 28 ist vorzugsweise in Form eines Fluidspenders ausgebildet, wobei ein Kopf **30** in Richtung der Längsachse **32** des Dermabrasionswerkzeugs 10, d.h. in Richtung eines Pfeils **34,** gegen eine Federkraft betätigbar ist, um durch einen Fluidauslass **36** im Dermabrasionswerkzeug 10 gespeichertes Fluid auszugeben. Das Fluid kann vor oder nach der Behandlung von Haut mit einem Dermabrasionsbereich 14 auf die Haut gegeben werden. Um die Pumpe 28 vor einer ungewünschten Betätigung zu schützen - beispielsweise wenn sich das Dermabrasionswerkzeug 10 in einer Handtasche befindet - kann das Dermabrasionswerkzeug 10 eine Kappe **38** aufweisen, die auf der Pumpe 28 anordenbar ist.

**Figur 5** zeigt einen Dermabrasionskörper **40** des Dermabrasionswerkzeugs 10 gemäß Figur 4 in Alleinstellung. Der Dermabrasionskörper 40 weist das Handteil 12 und den Dermabrasionsbereich 14 auf. Weiterhin kann der Dermabrasionskörper 40 einen Gewindeanschluss **42** aufweisen. Der Gewindeanschluss 42 ist vorzugsweise einteilig mit dem Handteil 12 ausgebildet. Der Gewindeanschluss 42 weist ein Gewinde **44** - hier in Form eines Außengewindes - auf. Hierdurch kann der Dermabrasionskörper 40 mittels einer Gewindeverbindung mit der Pumpe 28 (siehe Figur 4) reversibel lösbar verbunden werden. Alternativ dazu kann eine nicht zerstörungsfrei lösbare Verbindung zwischen dem Dermabrasionskörper 40 und der Pumpe 28 vorgesehen sein, um für den Anwender sichtbar zu machen, ob es sich bei dem Dermabrasionswerkzeug 10 um ein original hergestelltes Dermabrasionswerkzeug handelt oder um ein wieder befülltes. Der Dermabrasionskörper 40, insbesondere das Handteil 12, weist einen Hohlraum 22 auf. Der Hohlraum 22 ist vorzugsweise zur Aufnahme eines Fluids ausgebildet, das über eine Öffnung **46** in die Pumpe 28 (siehe Figur 4) gelangen und dort über die Fluidauslass 36 ausgegeben werden kann.

**Figur 6** zeigt ein weiteres Dermabrasionswerkzeug 10. Das Dermabrasionswerkzeug 10 gemäß Figur 6 ist identisch zum Dermabrasionswerkzeug 10 gemäß Figur 4 ausgebildet. Allerdings weist ein Dermabrasionsbereich 14 des Dermabrasionswerkzeugs 10 gemäß Figur 6 eine Durchgangsausnehmung 20 auf, die einen Hohlraum (vgl. Hohlraum 11 gemäß Figur 5) mit der Umgebung 24 verbindet. Weiterhin weist eine Pumpe 28 des Dermabrasionswerkzeugs 10 gemäß Figur 6 keinen Auslass auf, so dass bei einer Betätigung der Pumpe 28 Fluid **48** aus dem Hohlraum des Dermabrasionswerkzeugs 10 durch die Durchgangsausnehmung 20 austritt. Der Austritt des Fluids 48 erfolgt somit im Dermabrasionsbereich 14. Bei dieser besonders vorteilhaften Ausführungsform des Dermabrasionswerkzeugs 10 kann das Fluid 48 des Dermabrasionswerkzeugs 10 somit unmittelbar während der Hautbehandlung im zu behandelnden bzw. im behandelten Bereich appliziert werden. Bevorzugt ist dabei die Durchgangsausnehmung 20 im Bereich der Längsachse 32 des Dermabrasionswerkzeugs 10 ausgebildet.

Unter Vornahme einer Zusammenschau aller Figuren der Zeichnung betrifft die Erfindung zusammenfassend ein Dermabrasionswerkzeug 10 zum Abreiben von Haut. Hierzu weist das Dermabrasionswerkzeug 10 einen Dermabrasionsbereich 14 mit Reibvorsprüngen 18 auf. Die Reibvorsprünge 18 können in Form von Partikeln ausgebildet sein, die in einer Trägermatrix 16 des Dermabrasionsbereich 14 eingebunden sind. Die Trägermatrix 16 ist einstückig mit einem Handteil 12 ausgebildet. Am Handteil 12 kann eine Pumpe 28 angeordnet sein, um Fluid 48 aus einem Hohlraum 22 des Dermabrasionswerkzeugs 10 auszugeben.

Trägermatrix 16 und Handteil 12 sind vorzugsweise Teil eines einstückigen Dermabrasionskörpers 40. Der Dermabrasionskörper 40 ist dabei insbesondere aus Glas, Plexiglas (Plastik) oder Edelmetall ausgebildet. In das Glas können Partikel des Dermabrasionsbereichs 14 eingebrannt sein.

## Patentansprüche

1. Stabförmiges Dermabrasionswerkzeug (10) mit einem Handteil (12) zum Halten und Führen des Dermabrasionswerkzeugs (10) und einem an einem axialen Ende des Dermabrasionswerkzeugs (10) vorgesehenen Dermabrasionsbereich (14), wobei der Dermabrasionsbereich (14) eine Trägermatrix (16) aufweist, an der mehrere Reibvorsprünge (18) zur mechanischen Hautbehandlung angeordnet oder ausgebildet sind, wobei die Reibvorsprünge (18) jeweils eine maximale Querschnittsbreite zwischen 0,5 µm und 500 µm aufweisen, wobei die Trägermatrix (16) einteilig mit dem Handteil (12) ausgebildet ist, wobei das Handteil (12) einen Hohlraum (22) und eine mit dem Hohlraum (22) verbundene Öffnung (46) zur Befüllung des Hohlraums (22) mit einem Fluid (48) aufweist, wobei das Dermabrasionswerkzeug (10) eine manuelle Pumpe (28) aufweist und die Pumpe (28) fluidisch mit dem Hohlraum (22) verbunden ist
**dadurch gekennzeichnet dass** der Dermabrasionsbereich (14) in Form einer Halbkugel ausgebildet ist und bei dem Dermabrasionswerkzeug (10) das Handteil (12) und die Trägermatrix (16) aus Glas ausgebildet sind und die in Form von Partikeln ausgebildeten Reibvorsprünge (18) teilweise in die Trägermatrix (16) eingebettet und in die Oberfläche der Trägermatrix (16) aus Glas eingebrannt sind.

2. Dermabrasionswerkzeug nach Anspruch 1, bei dem die Reibvorsprünge (18) jeweils eine maximale Querschnittsbreite zwischen 20 µm und 350 µm aufweisen.

3. Dermabrasionswerkzeug nach einem der vorhergehenden Ansprüche, bei dem der Dermabrasionsbereich (14) eine konvexe oder konkave Grundform aufweist.

4. Dermabrasionswerkzeug nach einem der vorhergehenden Ansprüche, bei dem der Hohlraum (22) zumindest teilweise mit einem Fluid (48) gefüllt ist, wobei das Fluid (48) eine Säure, eine Lauge und/oder ein Medikament aufweist.

5. Dermabrasionswerkzeug nach Anspruch 4, bei dem das Fluid (48) ein Pathogen aufweist.

6. Dermabrasionswerkzeug nach einem der vorhergehenden Ansprüche, bei dem der Dermabrasionsbereich (14) eine Durchgangsausnehmung (20, 20a, 20b) von der Umgebung (24) des Dermabrasionswerkzeugs (10) zum Hohlraum (22) aufweist.

7. Dermabrasionswerkzeug nach einem der vorhergehenden Ansprüche, bei dem der Hohlraum (22) über ein Rückschlagventil (26) fluidisch mit der Umgebung (24) des Dermabrasionswerkzeugs (10) in Verbindung steht, wobei die Durchflussrichtung des Rückschlagventils (26) vom Hohlraum (22) in die Umgebung (24) und die Sperrrichtung des Rückschlagventils (26) von der Umgebung (24) in den Hohlraum (22) führt.

8. Dermabrasionswerkzeug nach einem der vorhergehenden Ansprüche, bei dem das Handteil (12) ein erstes Gewinde (44) aufweist, das mit einem zweiten Gewinde der Pumpe (28) verbunden ist.

## Claims

1. Stick-shaped dermabrasion tool (10) comprising a hand part (12) for holding and guiding the dermabrasion tool (10) and comprising a dermabrasion region (14) provided at an axial end of the dermabrasion tool (10), the dermabrasion region (14) comprising a carrier matrix (16) on which a plurality of abrasive projections (18) are arranged or formed for mechanical treatment of the skin, the abrasive projections (18) each having a maximum cross-sectional width between 0.5 µm and 500 µm, the carrier matrix (16) being formed in one piece with the hand part (12), the hand part (12) comprising a cavity (22) and an opening (46) connected to the cavity (22) for filling the cavity (22) with a fluid (48), the dermabrasion tool (10) comprising a manual pump (28) and the pump (28) being fluidically connected to the cavity (22),
**characterised in that**
the dermabrasion region (14) is in the form of a hemisphere and in the dermabrasion tool (10) the hand part (12) and the carrier matrix (16) are made of glass and the abrasive projections (18) in the form of particles are, in part, embedded in the carrier matrix (16) and baked into the surface of the carrier matrix (16) made of glass.

2. Dermabrasion tool according to claim 1, wherein the abrasive projections (18) each have a maximum cross-sectional width between 20 µm and 350 µm.

3. Dermabrasion tool according to either of the preceding claims, wherein the dermabrasion region (14) has a convex or concave basic shape.

4. Dermabrasion tool according to any one of the preceding claims, wherein the cavity (22) is filled at least in part with a fluid (48), the fluid (48) comprising an acid, an alkaline solution and/or medication.

5. Dermabrasion tool according to claim 4, wherein the fluid (48) comprises a pathogen.

6. Dermabrasion tool according to any one of the preceding claims, wherein the dermabrasion region (14) comprises a through-opening (20, 20a, 20b) from the surroundings (24) of the dermabrasion tool (10) to the cavity (22).

7. Dermabrasion tool according to any one of the preceding claims, wherein the cavity (22) is fluidically connected to the surroundings (24) of the dermabrasion tool (10) via a non-return valve (26), the flow direction of the non-return valve (26) leading from the cavity (22) into the surroundings (24) and the shut-off direction of the non-return valve (26) leading from the surroundings (24) into the cavity (22).

8. Dermabrasion tool according to any one of the preceding claims, wherein the hand part (12) comprises a first thread (44) which is connected to a second thread of the pump (28).

## Revendications

1. Outil de dermabrasion en forme de bâtonnet (10) comportant une pièce à main (12) pour tenir et guider l'outil de dermabrasion (10) et une zone de dermabrasion (14) prévue à une extrémité axiale de l'outil de dermabrasion (10), la zone de dermabrasion (14) présentant une matrice de support (16) sur laquelle sont disposées ou formées plusieurs saillies de friction (18) pour le traitement mécanique de la peau, les saillies de friction (18) ayant chacune une largeur en section transversale maximale comprise entre 0,5 µm et 500 µm, la matrice de support (16) étant formée d'un seul tenant avec la pièce à main (12), la pièce à main (12) présentant une cavité (22) et une ouverture (46) reliée à la cavité (22) pour remplir la cavité (22) avec un fluide (48), l'outil de dermabrasion (10) présentant une pompe manuelle (28) et la pompe (28) étant reliée fluidiquement à la cavité (22),
**caractérisé en ce que**
la zone de dermabrasion (14) est réalisée en forme de demi-sphère et la pièce à main (12) et la matrice de support (16) de l'outil de dermabrasion (10) sont réalisées en verre et les saillies de friction (18) réalisées sous la forme de particules sont partiellement noyées dans la matrice de support (16) et fixées par cuisson dans la surface de la matrice de support (16) en verre.

2. Outil de dermabrasion selon la revendication 1, dans lequel les saillies de friction (18) présentent chacune une largeur en section transversale maximale comprise entre 20 µm et 350 µm.

3. Outil de dermabrasion selon l'une des revendications précédentes, dans lequel la zone de dermabrasion (14) présente une forme de base convexe ou concave.

4. Outil de dermabrasion selon l'une des revendications précédentes, dans lequel la cavité (22) est au moins partiellement remplie d'un fluide (48), le fluide (48) présentant un acide, une base et/ou un médicament.

5. Outil de dermabrasion selon la revendication 4, dans lequel le fluide (48) présente un agent pathogène.

6. Outil de dermabrasion selon l'une des revendications précédentes, dans lequel la zone de dermabrasion (14) présente un évidement traversant (20, 20a, 20b) allant de l'environnement (24) de l'outil de dermabrasion (10) vers la cavité (22).

7. Outil de dermabrasion selon l'une des revendications précédentes, dans lequel la cavité (22) est en communication fluidique avec l'environnement (24) de l'outil de dermabrasion (10) par l'intermédiaire d'un clapet anti-retour (26), le sens de passage du clapet anti-retour (26) conduisant de la cavité (22) dans l'environnement (24) et le sens de blocage du clapet anti-retour (26) de l'environnement (24) dans la cavité (22).

8. Outil de dermabrasion selon l'une des revendications précédentes, dans lequel la pièce à main (12) présente un premier filetage (44) qui est relié à un deuxième filetage de la pompe (28).
